# EUROPEAN PATENT APPLICATION

(11) **EP 3 960 861 A1**
(43) Date of publication of application: **02.03.2022**
(21) Application number: 20193726.5
(22) Date of filing: 31.08.2020
(51) Int. Cl.: C12N 15/115, A61K 31/7115, C12Q 1/68, G01N 33/68, A61P 37/06

(54) **APTAMERS THAT TARGET CXCL9**

(71) Applicant: Rheinische Friedrich-Wilhelms-Universität Bonn, 53113 Bonn (DE); Leibniz Universität Hannover, 30167 Hannover (DE)
(72) Inventor: MAYER, Günter, 53123 Bonn (DE); SIEGL, Julia, 53119 Bonn (DE); BLUME, Cornelia, 30900 Wedemark (DE); PHUNG, Linh, 30419 Hannover (DE)
(74) Representative: Michalski Hüttermann & Partner Patentanwälte mbB

(57) **Abstract**

The present invention relates to an aptamer comprising a nucleotide sequence SEQ ID NO: 1, preferably comprising or consisting of a nucleotide sequence SEQ ID NO: 4-6. The invention further relates to a composition comprising the aptamer, and the use of the aptamer as a medicament or a diagnostic reagent, particularly for use in the detection or diagnosing of a rejection of a renal allograft.

SEQUENCE LISTING

<110>  Rheinische Friedrich-Wilhelms-Universität Bonn, Leibniz
       Universität Hannover

<120>  Aptamers that target CXCL9

<130>  UD 41404/SAM

<160>  17

<170>  PatentIn version 3.3

<210>  1
<211>  25
<212>  DNA
<213>  Artificial

<220>
<223>  aptamer


<220>
<221>  misc_feature
<222>  (1)..(1)
<223>  n is T or EdU

<220>
<221>  misc_feature
<222>  (2)..(12)
<223>  n is a sequence of 9 or 11 contiguous nucleotides comprising at
       least 5 guanidine nucleotides

<220>
<221>  misc_feature
<222>  (14)..(14)
<223>  n is A or C

<220>
<221>  misc_feature
<222>  (17)..(17)
<223>  n is T or EdU

<220>
<221>  misc_feature
<222>  (19)..(19)
<223>  n is A or a deletion

<220>
<221>  misc_feature
<222>  (20)..(20)
<223>  n is C or G

<220>
<221>  misc_feature
<222>  (21)..(21)
<223>  n is T or EdU

<220>
<221>  misc_feature
<222>  (22)..(22)
<223>  n is C or a deletion

<220>
<221>  misc_feature
<222>  (23)..(23)
<223>  n is C or G

<220>
<221>  misc_feature
<222>  (24)..(24)
<223>  n is G or T or EdU

<220>
<221>  misc_feature
<222>  (25)..(25)
<223>  n is T or EdU

<400>  1
nnnnnnnnnn nngnccnann nnnnn                                          25


<210>  2
<211>  23
<212>  DNA
<213>  artificial

<220>
<223>  aptamer


<220>
<221>  misc_feature
<222>  (1)..(1)
<223>  n is T or EdU

<220>
<221>  misc_feature
<222>  (15)..(15)
<223>  n is T or EdU

<220>
<221>  misc_feature
<222>  (19)..(19)
<223>  n is T or EdU

<220>
<221>  misc_feature
<222>  (23)..(23)
<223>  n is T or EdU

<400>  2
ngggcaaagg gcccnaagnc cgn                                            23


<210>  3
<211>  23
<212>  DNA
<213>  artificial

<220>
<223>  aptamer


<220>
<221>  misc_feature

<222>  (1)..(1)
<223>  n is T or EdU

<220>
<221>  misc_feature
<222>  (17)..(17)
<223>  n is T or EdU

<220>
<221>  misc_feature
<222>  (20)..(20)
<223>  n is T or EdU

<220>
<221>  misc_feature
<222>  (22)..(23)
<223>  n is T or EdU

<400>  3
ncacgggcgg gcgaccnacn gnn                                              23


<210>  4
<211>  84
<212>  DNA
<213>  Artificial

<220>
<223>  aptamer


<220>
<221>  misc_feature
<222>  (31)..(31)
<223>  n is T or EdU

<220>
<221>  misc_feature
<222>  (45)..(45)
<223>  n is T or EdU

<220>
<221>  misc_feature
<222>  (49)..(49)
<223>  n is T or EdU

<220>
<221>  misc_feature
<222>  (53)..(53)
<223>  n is T or EdU

<400>  4
cacgacgcaa gggaccacag ggagggaggg ngggcaaagg gcccnaagnc cgnaacaaaa      60

acacagcacg acaccgcaga ggca                                             84


<210>  5
<211>  84
<212>  DNA
<213>  Artificial

<220>

<223>  aptamer

<220>
<221>  misc_feature
<222>  (28)..(28)
<223>  n is T or EdU

<220>
<221>  misc_feature
<222>  (44)..(44)
<223>  n is EdU

<220>
<221>  misc_feature
<222>  (47)..(47)
<223>  n is EdU

<220>
<221>  misc_feature
<222>  (49)..(50)
<223>  n is EdU

<400>  5
cacgacgcaa gggaccacag gagagacnca cgggcgggcg accnacngnn cagcccagac      60

cgacagcacg acaccgcaga ggca                                              84


<210>  6
<211>  84
<212>  DNA
<213>  Artificial

<220>
<223>  aptamer

<400>  6
cacgacgcaa gggaccacag ggagggaggg tgggcaaagg gccctaagtc cgtaacaaaa      60

acacagcacg acaccgcaga ggca                                              84


<210>  7
<211>  84
<212>  DNA
<213>  Artificial

<220>
<223>  aptamer


<220>
<221>  misc_feature
<222>  (44)..(44)
<223>  n is EdU

<220>
<221>  misc_feature
<222>  (47)..(47)
<223>  n is EdU

<220>

<221>  misc_feature
<222>  (49)..(50)
<223>  n is EdU

<400>  7
cacgacgcaa gggaccacag gagagactca cgggcgggcg accnacngnn cagcccagac     60

cgacagcacg acaccgcaga ggca                                            84


<210>  8
<211>  38
<212>  DNA
<213>  Artificial

<220>
<223>  aptamer


<220>
<221>  misc_feature
<222>  (6)..(6)
<223>  n is T or EdU

<220>
<221>  misc_feature
<222>  (20)..(20)
<223>  n is T or EdU

<220>
<221>  misc_feature
<222>  (24)..(24)
<223>  n is T or EdU

<220>
<221>  misc_feature
<222>  (28)..(28)
<223>  n is T or EdU

<400>  8
gagggngggc aaagggcccn aagnccgnaa caaaaaca                             38


<210>  9
<211>  42
<212>  DNA
<213>  Artificial

<220>
<223>  aptamer


<220>
<221>  misc_feature
<222>  (7)..(7)
<223>  n is T or EdU

<220>
<221>  misc_feature
<222>  (23)..(23)
<223>  n is EdU

<220>

<221>  misc_feature
<222>  (26)..(26)
<223>  n is EdU

<220>
<221>  misc_feature
<222>  (28)..(29)
<223>  n is EdU

<400>  9
agagacncac gggcgggcga ccnacngnnc agcccagacc ga                          42


<210>  10
<211>  84
<212>  DNA
<213>  Artificial

<220>
<223>  aptamer



<220>
<221>  misc_feature
<222>  (22)..(63)
<223>  N is a, c, g or EdU

<400>  10
cacgacgcaa gggaccacag gnnnnnnnnn nnnnnnnnnn nnnnnnnnnn nnnnnnnnnn      60

nnncagcacg acaccgcaga ggca                                              84


<210>  11
<211>  21
<212>  DNA
<213>  Artificial

<220>
<223>  primer

<400>  11
cacgacgcaa gggaccacag g                                                 21


<210>  12
<211>  21
<212>  DNA
<213>  Artificial

<220>
<223>  primer

<400>  12
cacgacgcaa gggaccacag g                                                 21


<210>  13
<211>  21
<212>  DNA
<213>  Artificial

<220>

<223>  primer

<400>  13
tgcctctgcg gtgtcgtgct g                                                21


<210>  14
<211>  84
<212>  DNA
<213>  Artificial

<220>
<223>  aptamer


<220>
<221>  misc_feature
<222>  (29)..(29)
<223>  n is T or EdU

<220>
<221>  misc_feature
<222>  (36)..(36)
<223>  n is T or EdU

<220>
<221>  misc_feature
<222>  (45)..(45)
<223>  n is T or EdU

<220>
<221>  misc_feature
<222>  (54)..(54)
<223>  n is T or EdU

<400>  14
cacgacgcaa gggaccacag gaggagagna ggcganacac gacgnagcgc aganaggacc      60

aagcagcacg acaccgcaga ggca                                             84


<210>  15
<211>  84
<212>  DNA
<213>  Artificial

<220>
<223>  aptamer


<220>
<221>  misc_feature
<222>  (27)..(27)
<223>  n is EdU

<220>
<221>  misc_feature
<222>  (37)..(37)
<223>  n is EdU

<220>
<221>  misc_feature
<222>  (41)..(41)

<223>  n is EdU

<220>
<221>  misc_feature
<222>  (48)..(48)
<223>  n is EdU

<220>
<221>  misc_feature
<222>  (59)..(59)
<223>  n is EdU

<400>  15
cacgacgcaa gggaccacag ggcacgngcg aggcgcnaca ncaccggngc aagcaccgng     60

caacagcacg acaccgcaga ggca                                            84


<210>  16
<211>  15
<212>  DNA
<213>  Artificial

<220>
<223>  aptamer


<220>
<221>  misc_feature
<222>  (5)..(5)
<223>  n is a, c, g, t or EdU

<220>
<221>  misc_feature
<222>  (6)..(6)
<223>  n is EdU

<220>
<221>  misc_feature
<222>  (9)..(9)
<223>  n is EdU

<220>
<221>  misc_feature
<222>  (11)..(12)
<223>  n is EdU

<400>  16
cgacnnacng nncag                                                      15


<210>  17
<211>  43
<212>  DNA
<213>  Artificial

<220>
<223>  click competitor


<220>
<221>  misc_feature
<222>  (1)..(42)

<223>  n is a, c, g or EdU

<400>  17
nnnnnnnnnn nnnnnnnnnn nnnnnnnnnn nnnnnnnnnn nna                    43

## Description

The present invention relates to aptamers that target CXCL9. These aptamers are particularly useful in the detection of renal transplant rejection.

Kidney transplantation is a curative and life-saving therapy for end-stage renal disease which has become a standard in industrialised countries. The major limiting factor for transplant survival and success of kidney transplantation is transplant rejection. Rejection of renal allografts is a common poster-surgery risk factor. If diagnosed early, the successful treatment is promising. The standard for diagnosing transplant rejection is made by biopsy. As the procedure of renal transplant biopsies is afflicted with serious clinical risks and side effects it cannot be applied frequently in clinical routine. Therefore numerous approaches for non-invasive diagnostic strategies such as the screening of proteins as markers for detection of transplant rejection have been established.

The pro-inflammatory chemokine receptor ligand 9 CXCL9 (CXCL 9) is one early onset marker for the rejection of the donor organ. In search for highly sensitive diagnostic tools which can be used as an early POC (point of care) test, aptamers in contrast to most antibodies have a high thermostability and no batch-to-batch variations and thus are an interesting tool for diagnostic tests. For example Ferguson et al., Sci Transl Med. 2013, 5(213), 213ra165, suggest to use an aptamer-based detector for monitoring levels of the chemokine CXCL9, which is associated with nephropathy, acute graft rejection and graft failure, to enhance therapeutic outcome for patients undergoing organ transplantation.

Aptamers are single chained nucleic acids, folding into well-defined three-dimensional shapes based on which they recognise target structures with high affinity and specificity. Aptamers of either single-stranded DNA or RNA that specifically bind to a target ligand are produced by a technique denoted Systematic Evolution of Ligands by Exponential Enrichment (SELEX). The SELEX method is for example described by C. Tuerk and L. Gold "Systematic Evolution of Ligands by Exponential Enrichment: RNA Ligands to bacteriophage T4 DNA polymerase", Science 1990, 249, 505-510. In the SELEX method, a candidate mixture of single stranded nucleic acids having regions of randomized sequence is contacted with a target structure and those nucleic acids having an increased affinity to the target are selected and amplified. After several iterations a nucleic acid with high affinity to the target is obtained. One drawback of the selection of conventional aptamers is their limited chemical diversity, hence, targeting of difficult molecules can be challenging. Further, the selection of aptamers that are chemically modified via so-called multimodal click-SELEX (Systematic Evolution of Ligands by Exponential Enrichment) is known.

Therefore, the object underlying the present invention was to provide aptamers that are able to target CXCL9.

The problem is solved by an aptamer comprising a nucleotide sequence 5'-N₁N₂GN₃CCN₁AN₄N₅N₁N₆N₇N₈N₁-3' (SEQ ID NO: 1) or pharmaceutically acceptable salts thereof, wherein:
- N₁: represents T or 5-ethynyl-2'-deoxyuridine (EdU) which is modified with a side chain selected from the group comprising indole, benzofurane, naphthalene, phenol, benzothiophene, guanidine and benzyl,
- N2: represents a sequence of 9 or 11 contiguous nucleotides comprising at least 5 guanidine nucleotides,
- N₃: represents A or C,
- N₄: represents A or a deletion,
- N₅: represents C or G,
- N₆: represents C or a deletion,
- N₇: represents C or G,
- N₈: represents G or N₁.

Surprisingly, aptamers could be selected that recognise the chemokines CXCL9 and CXCL11 with high affinity and specificity. The aptamers further were shown to be able to bind to the recombinant and to the endogenous CXCL9. The aptamers thus are usable to detect CXCL9 in biological samples. The chemokine CXCL9 is an early onset marker for organ rejection in plasma and urine of kidney transplant patients. The aptamers providing high affinity and specificity for CXCL9 and CXCL11 thus allow a use as non-invasive biomarkers for diagnosing transplant rejection in kidney transplant recipients. The aptamers are usable in diagnostic tests for the detection of a kidney rejection. Such tests can improve early diagnosis-making in transplant rejection after kidney transplantation and help predict long-term outcome after kidney transplantation. Generally, aptamer represents a cost-effective tool as aptamers can be synthesised chemically.

As used herein, the term "aptamer" refers to a single-stranded oligonucleotide that recognises its target with high specificity and binds to the target with high affinity in the low nanomolar range. The aptamer can be provided in the form of a single-stranded DNA or RNA molecule. As will be obvious to a person of ordinary skills in the art, if the nucleic acid is an RNA molecule the thymidine or "T" in the nucleotide sequence is to be read as meaning "U" or uridine. Preferably, the aptamer comprises a deoxyribonucleotide sequence. DNA aptamers can exhibit better stability. The nucleotides may comprise a chemical modification such as an aromatic group.

The aptamers comprise a G-rich sequence comprising the sequence of 9 or 11 contiguous nucleotides N₂, followed by a shorter motif comprising three or four thymidines (Ts) or modified 5-ethynyl-2'-deoxyuridines (EdUs) that were found to be important for binding to CXCL9. The G-rich sequence N₂ comprises at least 5 further guanidine nucleotides. In case N2 represents a sequence of 9 contiguous nucleotides N₂ may comprise 5 further guanidine nucleotides. In case N₂ represents a sequence of 11 contiguous nucleotides N₂ may comprise 6 further guanidine nucleotides. Preferably, the aptamer comprises two triplicates of guanidines, either comprised in contiguous nucleotides of N₂ or including the G following N₂. The further nucleotides of the sequence of 9 or 11 contiguous nucleotides of N₂ may be selected from A, C, G or T, preferably from A and C.

The aptamer may comprise a nucleotide sequence 5'-N₁GGGCAAAGGGCCCN₁AAGN₁ CCGNi-3' (SEQ ID NO: 2) or pharmaceutically acceptable salts thereof, wherein N₁ represents T or EdU which is modified with a side chain selected from the group comprising indole, phenol, guanidine and benzyl. Or the aptamer may comprise a nucleotide sequence 5'-N₁CACGGGCGGGCGACCN₁ACN₁GN₁N₁-3' (SEQ ID NO: 3) wherein N₁ represents T or EdU which is modified with an aromatic group selected from the group comprising indole, benzofurane, naphthalene, phenol and benzothiophene.

EdU (5-ethynyl-2'-deoxyuridine) is a nucleoside analog of thymidine, carrying an ethynyl group. EdU is incorporated into replicating DNA instead of its natural analog thymidine. The ethynyl groups of the resulting ethynyl-functionalized DNA can subsequently be modified via Cu(I)-catalyzed click chemistry with azides of various compounds, such as with a side chain selected from indole, benzofurane, naphthalene, phenol or benzothiophene benzyl, or guanidine.

In embodiments, the aptamer comprises or consists of a nucleotide sequence 5'-CACGACGCAAGGGACCACAGGGAGGGAGGGN₁GGGCAAAGGGCCCN₁A AGN₁CCGN₁AACAAAAACACAGCACGACACCGCAGAGGCA-3' (SEQ ID NO: 4) or pharmaceutically acceptable salts thereof, wherein N₁ represents T or EdU which is modified with a side chain selected from the group comprising indole, phenol, guanidine and benzyl. The aptamers were shown to recognise the chemokines CXCL9 and CXCL11 with high affinity and specificity. The aptamers of the sequence of SEQ ID NO: 4 were shown to provide similar affinity for CXCL9 and CXCL11, but no binding to CXCL10.

The aptamer of SEQ ID NO: 4 was shown to be able to bind to CXCL9 and CXCL11 either as non-modified, conventional DNA or modified with different side chains. The aptamer of SEQ ID NO: 4 thus may comprise EdU moieties modified with side chains selected from indole, phenol, guanidine or benzyl or may be used as conventional DNA. In preferred embodiments, the the aptamer comprises or consists of a nucleotide sequence 5'-CACGACGCAAGGGACCACAGGGAGGGAGGGTGGGCAAAGGGCCCTA AGTCCGTAACAAAAACACAGCACGACACCGCAGAGGCA-3' (SEQ ID NO: 6) or of SEQ ID NO: 4 wherein N₁ represents EdU which is modified with with side chains selected from indole, phenol, guanidine or benzyl, or pharmaceutically acceptable salts thereof. In preferred embodiments, the aptamer comprises or consists of a nucleotide sequence SEQ ID NO: 4 or pharmaceutically acceptable salts thereof, wherein N₁ represents EdU which is modified with indole. The aptamer of SEQ ID NO: 4 modified with indole residues was shown to provide enhanced binding abilities for CXCL9.

In further embodiments, the aptamer comprises or consists of a nucleotide sequence 5'-CACGACGCAAGGGACCACAGGAGAGACN₁CACGGGCGGGCGACCN'₁ACN'₁ GN'₁N'₁CAGCCCAGACCGACAGCACGACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene. The aptamer of SEQ ID NO: 5 was shown to bind to CXCL9 when modified with aromatic groups selected from indole, benzofurane, naphthalene, phenol or benzothiophene. The aptamer was shown to recognise the chemokine CXCL9 with high affinity and specificity, but no binding to CXCL10 was detected. In preferred embodiments, the aptamer comprising or consisting of a nucleotide sequence SEQ ID NO: 5 or pharmaceutically acceptable salts thereof is modified with an aromatic group selected from indole, benzofurane or naphthalene. Indole, benzofurane and naphthalene modifications provided enhanced binding to chemokine CXCL9.

The first N₁ member of SEQ ID NO: 5 may be selected from T or modified EdU. In embodiments, N₁ and N'₁ both represent EdU modified with an aromatic group. In embodiments, the aptamer may comprise or consist of a nucleotide sequence 5'-CACGACGCAAGGGACCACAGGAGAGACTCACGGGCGGGCGACCN'₁ACN'₁ GN'₁N'₁CAGCCCAGACCGACAGCACGACACCGCAGAGGCA-3' (SEQ ID NO: 7) or pharmaceutically acceptable salts thereof, wherein N'₁ represents EdU which is modified with an aromatic group selected from the group comprising indole, benzofurane, naphthalene, phenol and benzothiophene.

The aptamer further may comprise or consist of a nucleotide sequence 5'-GAGGGN₁GGGCAAAGGGCCCN₁AAGN₁CCGN₁AACAAAAACA-3' (SEQ ID NO: 8) or pharmaceutically acceptable salts thereof, wherein N₁ represents T or EdU which is modified with a side chain selected from the group comprising indole, phenol, guanidine and benzyl. Or the aptamer may comprise or consist of a nucleotide sequence 5'-AGAGACN₁CA CGGGCGGGCGACCN'₁ACN'₁GN'₁N'₁CAGCCCAGACCGA-3' (SEQ ID NO: 9) or pharmaceutically acceptable salts thereof, wherein N₁ represents T or EdU which is modified with an aromatic group selected from the group comprising indole, benzofurane, naphthalene, phenol and benzothiophene, and N'₁ represents EdU which is modified with an aromatic group selected from the group comprising indole, benzofurane, naphthalene, phenol and benzothiophene.

The aptamers also are usable in form of pharmaceutically acceptable salts thereof. The term "pharmaceutically acceptable salts" refers to salts prepared from pharmaceutically acceptable non-toxic bases or acids. A pharmaceutically acceptable salt can be conveniently prepared from pharmaceutically acceptable non-toxic bases, including inorganic bases and organic bases. Preferred salts derived from inorganic bases include ammonium, calcium, magnesium, potassium and sodium salts. Salts derived from pharmaceutically acceptable organic non-toxic bases include salts of primary, secondary, and tertiary amines, as well as cyclic amines. Preferably, the pharmaceutically acceptable salt is selected from the group of sodium or potassium salts. Also, calcium or magnesium salts can be preferred.

A further aspect relates to an aptamer comprising or consisting of a nucleotide sequence selected from the group of SEQ ID NO: 1-9, preferably from SEQ ID NO: 4-6, or pharmaceutically acceptable salts thereof for use as a medicament or a diagnostic reagent. For the description of the aptamers, reference is made to the description above. In preferred embodiments, the aptamer comprises or consists of a nucleotide sequence SEQ ID NO: 6 or SEQ ID NO: 4 wherein N₁ represents EdU which is modified with indole, phenol, guanidine and benzyl, preferably indole, or pharmaceutically acceptable salts thereof. In further preferred embodiments, the aptamer comprises or consists of a nucleotide sequence 5'-CACGACGCAAGGGACCACAGGAGAGACN₁CACGGGCGGGCGACCN'₁ACN'₁ GN'₁N'₁CAGCCCAGACCGACAGCACGACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene.

The aptamers advantageously are able to recognise chemokines CXCL9 with high affinity and in biological samples. This allows that the aptamer can be used for the detection of early onset marker for the rejection of a transplanted organ and suggests a use in the diagnosis of transplant rejection. The aptamers thus allow a use as non-invasive biomarkers for diagnosing transplant rejection in kidney transplant recipients. The aptamers particularly are usable as a diagnostic reagent in tests for the detection of a kidney rejection. Hence, the aptamer is useful as a medicament or a diagnostic reagent.

In preferred embodiments, the aptamer is for use in the detection or diagnosing of a rejection of a renal allograft. The aptamer allows detecting binding to chemokines CXCL9 with high affinity and specificity in biological samples and thus a use in highly sensitive diagnostic tools to be used as an early POC (point of care) test. The aptamers may help to improve early diagnosis-making in intransplant rejection after kidney transplanation.

The aptamer may be used alone or in combination with further markers for pro-inflammatory chemokines such as antibodies. The aptamer may be bound or forming a complex, such as a sandwich complex with antibodies. The aptamer may be used with antibodies detecting CXCL9, or antibodies detecting further chemokines. In preferred embodiments, the aptamer is used in form a complex with an antibody against chemokines CXCL9. By detecting binding to chemokine CXCL9 via aptamer and antibody specificity of a test may be optimised.

For use as a medicament or a diagnostic reagent the aptamers can be used or included in a composition. For use as a medicament the aptamer can be used or included in a pharmaceutical composition.

For use as a diagnostic reagent the aptamer can be used or included in a diagnostic composition. The aptamers, for example, may be used as a diagnostic reagent in lateral flow assays or in enzyme-linked oligonucleotide assays (ELONA). Accordingly, a further aspect relates to a diagnostic composition comprising as an active ingredient an aptamer comprising or consisting of a nucleotide sequence selected from the group of SEQ ID NO: 1-9, preferably from SEQ ID NO: 4-6, or pharmaceutically acceptable salts thereof. For the description of the aptamers, reference is made to the description above. In preferred embodiments, the aptamer comprises or consists of a nucleotide sequence SEQ ID NO: 6 or SEQ ID NO: 4 wherein N₁ represents EdU which is modified with indole, phenol, guanidine and benzyl, preferably indole, or pharmaceutically acceptable salts thereof. In further preferred embodiments, the aptamer comprises or consists of a nucleotide sequence 5'-CACGACGCAAGGGACCACAG GAGAGACN₁CACGGGCGGGCGACCN'₁ACN'₁GN'₁N'₁CAGCCCAGACCGACAGCACG ACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene.

By detecting binding to chemokines CXCL9 and CXCL11, the composition particularly is usable in the detection or diagnosis of the rejection of the donor organ such as after kidney transplanation. In preferred embodiments, the diagnostic composition is for use in the detection or diagnosing of a rejection of a renal allograft. The diagnostic composition comprising the aptamers may help to improve early diagnosis-making in intransplant rejection after kidney transplanation.

When the aptamer is brought into contact with a sample, it will bind specifically to chemokines CXCL9 and CXCL11 present in the sample. For diagnostic purposes, the aptamer may comprise a labelling which provides that the bound aptamer can be detected by determining the presence or absence of a signal provided by the label. For example, the aptamer can be labelled with a fluorescent dye. A fluorescence-labelling, for example provided by a fluorescence dye, can provide a visualisation of the bound aptamer by fluorescence or laser scanning microscopy or flow cytometry. Further, particularly for detection purposes, the aptamer can be immobilised on conventional supports such as beads providing a tool for the detection of aptamer-bound chemokine and thus diagnosing possible rejection of renal allograft. Further, the aptamer can be biotinylated or coupled to streptavidin, avidin or neutravidin for use in the specific detection of chemokines.

Besides being useful for detecting or diagnosing rejection of a donor organ by recognising chemokines, the aptamers also are applicable for targeted therapies. Particularly for use as a medicament the aptamers can be used or included in a pharmaceutical composition. Accordingly, in another aspect the present invention relates to a pharmaceutical composition comprising as an active ingredient an aptamer comprising or consisting of a nucleotide sequence selected from the group of SEQ ID NO: 1-9, preferably from SEQ ID NO: 4-6, or pharmaceutically acceptable salts thereof. For the description of the aptamers, reference is made to the description above. In preferred embodiments, the aptamer comprises or consists of a nucleotide sequence SEQ ID NO: 6 or SEQ ID NO: 4 wherein N₁ represents EdU which is modified with indole, phenol, guanidine and benzyl, preferably indole, or pharmaceutically acceptable salts thereof. In further preferred embodiments, the aptamer comprises or consists of a nucleotide sequence 5'-CACGACGCAAGGGACCACAGGAGAGACNiCACGGGC GGGCGACCN'₁ACN'₁GN'₁N'₁CAGCCCAGACCGACAGCACGACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene.

The pharmaceutical composition comprising the aptamers can particularly be useful as a molecular vehicle for delivery of cargo such as anti-inflammatory agents to the chemokines for reducing inflammation and donor organ rejection. Compounds, drugs or effector molecules can be directly coupled to the aptamer, in a covalent or non-covalent fashion. Alternatively, the aptamer can be attached to the surface of a liposome containing an anti-inflammatory agent or to nanoparticles encapsulating an anti-inflammatory agent. The aptamers may provide a chemokine-specific drug delivery composition comprising an aptamer and an anti-inflammatory agent such as an anti-inflammatory drug.

The composition may be produced under sterile conditions using standard pharmaceutical techniques well known to those skilled in the art. For compositions convenient pharmaceutical media may be employed. For example, water, glycols, oils, alcohols and the like may be used to form liquid preparations such as solutions. The composition may comprise a pharmaceutical carrier, which can be, for example, a solid, liquid, or gas. Suitable carriers preferably are liquid and correspond to the substances ordinarily employed in formulation technology for pharmaceutical formulations.

The present invention also relates to the use of an aptamer comprising or consisting of a nucleotide sequence selected from the group of SEQ ID NO: 1-9, preferably from SEQ ID NO: 4-6, or pharmaceutically acceptable salts thereof, for the manufacture of a medicament or a diagnostic reagent. The diagnostic reagent particularly is for use in the detection or diagnosing of a rejection of a renal allograft. For the description of the aptamers, reference is made to the description above. In preferred embodiments, the aptamer comprises or consists of a nucleotide sequence SEQ ID NO: 6 or SEQ ID NO: 4 wherein N₁ represents EdU which is modified with indole, phenol, guanidine and benzyl, preferably indole, or pharmaceutically acceptable salts thereof. In further preferred embodiments, the aptamer comprises or consists of a nucleotide sequence 5'-CACGACGCAAGGGACCACAGGAGAGACNiCACGGGC GGGCGACCN'₁ACN'₁GN'₁N'₁CAGCCCAGACCGACAGCACGACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene.

A further aspect relates to an *in vitro* method of detecting or diagnosing a rejection of a renal allograft, the method comprising the step of detecting the binding of an aptamer comprising or consisting of a nucleotide sequence selected from the group of SEQ ID NO: 1-9, preferably from SEQ ID NO: 4-6, or pharmaceutically acceptable salts thereof, to CXCL9 or CXCL11 in a sample obtained from a subject. For the description of the aptamers, reference is made to the description above. In preferred embodiments, the aptamer comprises or consists of a nucleotide sequence SEQ ID NO: 6 or SEQ ID NO: 4 wherein N₁ represents EdU which is modified with indole, phenol, guanidine and benzyl, preferably indole, or pharmaceutically acceptable salts thereof. In further preferred embodiments, the aptamer comprises or consists of a nucleotide sequence 5'-CACGACGCAAGGGACCACAGGAGAGACN₁CACGGGCGGGCGACCN'₁ACN'₁ GN'₁N'₁CAGCCCAGACCGACAGCACGACACCGCAGAGGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and N'₁ represents EdU which is modified with an aromatic group selected from the group comprising indole, benzofurane, naphthalene, phenol and benzothiophene.

As used herein, the term "sample" refers to any material, which probably contains chemokines, particularly CXCL9 or CXCL11, including any liquid or fluid sample or solid material, particularly a sample derived from a biological source such as a patient or test subject. The sample may be derived from a biological source such as a renal allograft subject. The term sample particularly refers to biological material, for example cells or tissues, biological fluids or supernatants. The sample for example can comprise cells or a tissue specimen isolated from a cancer subject, preferably a human, for example, by surgical resection or biopsy. The sample can be a body fluid such as blood, serum, plasma, saliva, phlegm and urine. The sample preferably is selected from urine or serum of a renal allograft patient, preferably a human. The method comprises bringing the aptamer into contact with a sample, which probably contains chemokines, particularly CXCL9 or CXCL11. Determination of biomarkers in urine samples is commonly used for point of care testing.

Unless otherwise defined, the technical and scientific terms used herein have the same meaning as commonly understood by one of ordinary skill in the art to which this invention belongs.

The Examples, which follow serve to illustrate the invention in more detail but do not constitute a limitation thereof.

The figures show:
- Figure 1: Interaction analysis using flow cytometry of a starting library binding to CXCL9 immobilised on beads and after 11 or 13 selection cycles. 500 nM Cy-5 labelled DNA from the starting library (SL) as well as from selection cycle 11 and selection cycle 13 were incubated with CXCL9 immobilised on magnetic beads. DNA was either unmodified (E-dU) or click modified with indole (In), benzyl (Bn), phenol (Phe) or guanidine (Gua) azide.
- Figure 2: in figure 2A the frequency and in figure 2B fold change of selection cycle 11 and selection cycle 13 of sequence SEQ ID NO: 4 (G123), and in figure 2C the frequency and and in figure 2D fold change of selection cycle 11 and selection cycle 13 of sequence SEQ ID NO: 5 (129).
- Figure 3: Interaction analysis with flow cytometry. 500 nM Cy-5 labelled DNA from the starting library (SL), from selection cycle 11 (c11) and sequence SEQ ID NO: 4 (G123) were incubated with CXCL9 immobilised on magnetic beads. DNA was either conventional DNA, unmodified (E-dU) or click modified with indole (In), benzyl (Bn), phenol (Phe) or guanidine (Gua) azide.
- Figure 4: Influence of modification for aptamer SEQ ID NO: 5 (129) binding to CXCL9. Biotin labelled DNA was detected by colorimetric change using streptavidin-horseradish peroxidase (SA-HRP) A binding of 100 nM of aptamer SEQ ID NO: 5 (129) with 10 different modifications (or w/o = E) to CXCL9.
- Figure 5: Substitution of the EdU modifications with conventional Ts of aptamer SEQ ID NO: 5 (129). Biotin labelled DNA (100 nM) of 129 variants was detected by colorimetric change using streptavidin-horseradish peroxidase (SA-HRP).
- Figure 6: G-quadruplex characterisation of G123 sequence. Figure 6A shows results of a flow cytometer binding assay. Figure 6B shows a circular dichroism analysis of SEQ ID NO: 4 (G123) DNA in water and in PBS.
- Figure 7: Competition assay of the indole-modified aptamer of SEQ ID NO: 5 (129 In), unmodified aptamer of SEQ ID NO: 6 (G123 DNA), and indole-modified aptamer of SEQ ID NO: 4 (G123 In).
- Figure 8: Characterisation of CXCL9 binding in different buffer conditions. Figure 8A shows results of a flow cytometer binding assay of conventional aptamer of SEQ ID NO: 6 (G123 DNA), the aptamer of SEQ ID NO: 4 click-modified with indole azide (G123In-dU), and the aptamer of SEQ ID NO: 5 click-modified with indole azide (G123In-dU) (n=3-4, SD ± mean, binding normalized to binding in SELEX conditions (pH 6.5)). Figure 8B shows results of an ELONA binding assay of biotin labelled G123 (DNA or with indole (In) modification) and 129 (In) in either SELEX conditions or in human urine.
- Figure 9: Binding to endogenous CXCL9. Binding to native CXCL9 in cell supernatant (native), recombinant CXCL9 in control cell supernatant (rec) and control supernatant without CXCL9 (ctrl) with Cy5-labelled aptamer of SEQ ID NO: 5 click-modified with indole azide (129) is shown in Figure 9A, aptamer of SEQ ID NO: 6 (G123 DNA) in Figure 9B and aptamer of SEQ ID NO: 4 click-modified with indole azide (G123 In) in Figure 9C.
- Figure 10: sandwich complex

### Reagents and materials:

### Chemicals

All chemicals, unless otherwise stated, where purchased from Sigma-Aldrich (Munich, Germany). 5-Ethynyl-dUTP (EdU) and tris(3-hydroxypropyltriazolmethyl)amine (THPTA) were purchased from BaseClick (Neuried, Germany). M280 streptavidin magnetic beads, salmon sperm DNA, EZ-Link Sulfo-NHS-LC-Biotin and λ-exonuclease were obtained from Thermo Fisher Scientific (Darmstadt, Germany). Preparations of azides (indole, phenole, guanidine) was done according to the protocol described in Suzuki et al., "Rapid discovery of highly potent and selective inhibitors of histone deacetylase 8 using click chemistry to generate candidate libraries", J Med Chem, 2012. 55(22): p. 9562-75. Benzyl azide was purchased from Sigma Aldrich.

### Proteins and enzymes

Proteins and enzymes were purchased from the suppliers as given in the following table 1:

**Table 1: Proteins and enzymes:**

| Protein | Supplier |
|---|---|
| Human CXCL9 | Origene |
| BSA | AppliChem |
| Human CXCL10 | Cell guidance svstem |
| Human CXCL11 | Cell guidance system |
| Human CXCL1 | Cell guidance svstem |
| Human sCD25 | Antikörper online |
| Native human CRP | BioRad |
| Human CCL17 | BioLegend |
| Human CCL22 | BioLegend |
| Human CCL3 | Peprotech |
| HSA | Sigma-Aldrich |
| Human CXCL9/MIG Biotinylated Antibody | R&D System |
| PWO DNA Polymerase | Genaxxon |
| λ exonuclease | Thermo Fisher Scientific |

### Oligonucleotides

HPLC purified oligonucleotides were purchased from Ella Biotech (Planegg, Germany). Sequences of the oligonucleotides are given in the following table 2:

**Table 2: Sequences of oligonucleotides:**

| Oligo Name | Sequence |
|---|---|
| FT2-0.35 | CACGACGCAAGGGACCACAGG – N42 – CAGCACGACACCGCAGAGGCA (SEQ ID NO: 10) N=A:G:C:EdU (1:1:1:0.35) |
| Click competitor | N42-A (SEQ ID NO: 17) N=A:G:C:EdU (1:1:1:1) |
| FT2-Fw-Cy5 | Cy5-CACGACGCAAGGGACCACAGG (SEQ ID NO: 11) |
| FT2-Fw-Bio | Bio-CACGACGCAAGGGACCACAGG (SEQ ID NO: 12) |
| FT2-Rev-P | Phos-TGCCTCTGCGGTGTCGTGCTG (SEQ ID NO: 13) |
| G123 | CACGACGCAAGGGACCACAGGGAGGGAGGGNGGGCAAA GGGCCCNAAGNCCGNAACAAAAACACAGCACGACACCGC AGAGGCA (SEQ ID NO: 4) N=T/EdU |
| G123.sc | CACGACGCAAGGGACCACAGGAGGAGAGNAGGCGANACA CGACGNAGCGCAGANAGGACCAAGCAGCACGACACCGCA GAGGCA (SEQ ID NO: 14) N=T/EdU |
| 129 | CACGACGCAAGGACCACAGGAGAGACNCACGGCGGG CGACCNACNGNNCAGCCCAGACCGACAGCACGACACCGC AGAGGCA (SEQ ID NO: 5) N=EdU |
| I29.sc | CACGACGCAAGGGACCACAGGGCACGNGCGAGGCGCNA CANCACCGGNGCAAGCACCGNGCAACAGCACGACACCGC AGAGGCA (SEQ ID NO: 15) N=EdU |

### Methods

### Biotinylation:

500 µl CXCL9 (39 nmol) were mixed with 15.7 µl sulfo-NHS-LC-Biotin (156 nmol) and incubated for 30 min on ice followed by incubation for 25 min at RT. Afterwards biotinylated protein was purified using zeba spin desalting columns (Thermo Fisher Scientific) according to manufacturer instructions.

### Bead preparation:

10 mg of M280-streptavidin magnetic dynabeads were washed three times with 1000 µl PBS and resuspended in 1000 µl PBS. 500 µl were used as empty beads and 50 µl of biotinylated CXCL9 (66 µM) was added to the remaining beads. After incubation at 25°C and 1000 rpm the supernatant was discarded, and the beads were washed three times with 500 µL PBS. Supernatant of empty and CXCL9 beads was discarded and beads were resuspended with 1x SB1 (138 mM NaCl, 5 mM KCl, 1.5 mM KH₂PO₄, 8.1mM Na₂HPO₄, 170 mM urea, 7 mM ammonium acetate, 1 mM MgCl₂, 1 mM CaCl₂, 1.2 mg/mL BSA, 0.1 mg/mL salmon sperm DNA, pH 6.5) and stored at 4°C.

### Click reaction:

The click reaction was done as described by Pfeiffer et al., "Identification and characterization of nucleobase-modified aptamers by click-SELEX", Nat Protoc, 2018. 13(5): p. 1153-1180. Shortly, freshly prepared sodium ascorbate (25 mM), CuSO₄ (1 mM) and THPTA (4 mM) in 100µL ddH₂O are incubated for 10-15 min (catalyst solution). Afterwards, EdU-DNA, was clicked in a solution containing 1mM azide in DMSO (10% final), 1x phosphate buffer and 1x catalyst solution in a total of 100 µL ddH₂O. The mixture was incubated 15 min at 37°C and 650 rpm. Afterwards the samples were purified using Nucleospin Gel and PCR clean-up kit from Macherey-Nagel (Düren, Germany) according to the manufacturer's instructions.

### Polymerase chain reaction (PCR):

PCR was performed in a veriti 96 well thermo cycler (Applied Biosystem). All PCRs contained 0.5 µM of both forward and reverse primer, 250 µM d^{∗}NTP mix (with EdU instead of T), PWO DNA polymerase (Genaxxon) and the supplied buffer (containing 2mM MgSO₄). The samples were prepared on ice and the following cycling program was used Step 1: 2 min 95°C, Step2 (denaturing): 30 s 92°C, Step 3 (annealing):30 s 62°C, Step 4 (extension): 1 min 72°C, Step 5: 2 min 72°C, hold 10°C; Step 2-4 were repeated).

### Split and combine click-SELEX:

For the first selection round, 125 pmol FT2-0.35 library were independently click modified with In-, Bn-, Phe and Gua-dU. Modified DNA was pooled together and incubated with 50 µl CXCL9-magnetic beads in 1x SB1 for 30 min at 25°C and 1000 rpm. After washing with 1xSB1 the beads were incubated for 5 min at 95°C, supernatant was used as template for PCR in a total volume of 800 µL. Purified dsDNA was incubated with 3.5 µl λ-exonuclease (10 U/µl, ThermoFisher) in 1x λ-exonuclease buffer for 20 min at 37°C and 650 rpm. Samples were purified using Nucleospin Gel and PCR clean-up kit from Macherey-Nagel according to the manufacturer's instructions. Finally, purified ssDNA was aliquoted to four samples which are, together with 125 pmol click competitor, individually click modified.
To increase the selection pressure several steps were modified: (1) addition of click competitor starting at cycle 2 (125 pmol for each azide), (2) increase in washing time and volume, (3) addition and increase of dextran sulfate from cycle 7, (4) decrease in incubation time, (5) reducing the number of magnetic beads (cycle 3-6), (6) reduction of biotinylated CXCL9 for immobilisation (1/5^{th} to 1/1000^{th} of original CXCL9 concentration used for bead preparation).

### Flow cytometry

The binding interaction of Cy5 labelled DNA to protein immobilized or captured on magnetic beads was investigated with a FACSCanto II (BD Bioscience). In total a minimum of 30'000 events was recorded and the Cy5-fluorescence in the APC-A channel was analysed.

Binding interaction of selection cycles and single sequences
500 nM of Cy5 labelled DNA and 1.5 µL CXCL9 beads in 10 µL SB 1 were incubated for 30 min, 25°C and 1000 rpm. Afterwards, the beads were washed shortly in 100 µL 1x SB1 and resuspended in 100 µL 1x SB1 for flow cytometer analysis.

Competition assay
100 nM Cy5 labelled DNA and 1 µM unlabelled DNA were incubated with 1 µL CXCL9 magnetic beads in 10 µL 1x SB1. After incubation (30 min, 25°C, 1000 rpm) the beads were washed 1x short and 1x 3 min with 100 µL SB1 and resuspended in 100 µl 1xSB1 for flow cytometer analysis.

Pull down native CXCL9
50 µg M280 streptavidin magnetic beads were washed 3 times with 40 µL PBS. 0.24 µg of biotinylated human CXCL9 antibody (R&D System) was incubated with 50 µg beads in 20 µL PBS for 40 min, 25°C and 1000 rpm. The beads were washed three times with 40 µL PBS, resuspended in 10 µL PBS + 1% BSA and used directly. Recombinant CXCL9 was diluted in control supernatant w/o CXCL9 to 11 ng/ml. 100 µl cell supernatant containing endogenous CXCL9 (11 ng/ml), control supernatant with and without CXCL9 were incubated with 1 µL of antibody beads for 30 min, 25°C and 1000 rpm. After, beads were washed 1x short and 1x 3min with 100 µl PBS + 1% BSA. 40 µL 150 nM Cy5-DNA in 1x SB 1 were incubated with beads for 30 min, 25°C, 1000 rpm. Without washing, the bead complex is analysed by flow cytometry. Binding of 150 nM Cy-5 DNA to antibody beads was used as background and was thus subtracted from samples.

Binding in different buffer conditions
CXCL9 beads were 100 nM Cy5 labelled DNA and 1.5 µl CXCL9 beads were incubated in 40µL either 1x SB1 (without ammonium acetate and urea) or in 1x SB1 w/o K⁺ (10 mM H₃PO₄ + NaOH, 1.2 mg/ml BSA, 0.1 mg/ml salmon sperm DNA) for 30 min at 25°C and 1000 rpm. Beads are directly analysed without washing steps.
pH of SB1 was adjusted accordingly for binding at different pH values.

### Next-generation sequencing (NGS):

NGS samples were prepared as described in Tolle et al. "Preparation of SELEX Samples for Next-Generation Sequencing", Methods Mol Biol, 2016. 1380: p. 77-84 and were measured on a Illumina HiSeq1500 platform. Shortly, PCR with index primers was done using canonical nucleotides, and thus replacing the site of modification with a T. PCR product was purified using Nucleospin Gel and PCR clean-up kit from Macherey-Nagel (Düren, Germany) according to the manufacturer's instructions. 83 ng of each purified DNA waa pooled, and adapter sequence was added by enzymatic ligation using TruSeq DNA PCR-Free Sample Preparation Kit LT (Illumina). After DNA agarose purification and Nucleospin Gel and PCR clean-up kit from Macherey-Nagel (Düren, Germany) clean up, the DNA was eluted in resuspension buffer (TruSeq DNA PCR-Free Sample Preparation Kit LT (Illumina)).
Prior sequencing the DNA was validated and quantified using KAPA library quantification kit (Sigma-Aldrich). Illumina sequencing was performed with 75 bp single end sequencing. Analysis of raw data was done using AptaNext software (Laura Lledo Byrant).

### (Enzyme linked oligonucleotide assay) ELONA

20 µl of 1 µg/ml protein in bicarbonate/carbonate buffer pH 9.6 was coated overnight at 4°C on 96 well half area plates (Greiner Bio). After washing three times with 100 µL WB1 (PBS+ 0.05% Tween 20), wells were blocked with 1% BSA in PBS for 2h at RT with slight agitation. Empty wells were also blocked for later subtraction of background binding. Wells were washed once with 100 µL WB2 (138 mM NaCl, 5 mM KCl, 1.5 mM KH₂PO₄, 8.1mM Na₂HPO₄, 170 mM urea, 7 mM ammonium acetate, 1 mM MgCl₂, 1 mM CaCl₂, pH 6.5).
DNA was diluted to respective concentration in SB1 and 20 µL is transferred onto the wells in duplicates (protein and BSA wells). After incubation of 30 min at RT and slight agitation, the wells were washed two times with 100 µL WB 1 and are incubated with 20 µL of streptavidin-horseradish peroxidase (SA-HRP) solution (1:1000 in PBS, GE Healtcare). After 30 min incubation at RT the wells were washed again twice with 100 µL WB 1. Finally, the complex was incubated with 1-Step ABTS (ThermoFisher) for 15-40 min at RT, slight agitation. Absorbance at 405 nm was read out with a Tecan Nanoquant plate reader. Binding of DNA to BSA background was subtracted from binding of DNA to protein sample.
The equilibrium dissociation constant was calculated with non-linear regression assuming one site specific binding model with GraphPad Prism 6.01 (Graph Pad Software, La Jolla, USA)

### Example 1

Selection of aptamers using multimodal split and combine click-SELEX

For use in the multimodal click-SELEX method, the non-natural nucleotide EdU, which was incorporated into the DNA library, was modified with copper (I) alkine-azide cycloaddition using the CuAAC or "click" reaction as described above with different functionalisations, 3-ethyl-1H-indole (In), 1-methyl-benzene (Bn), 4-ethyl-phenol (Phe) and N-ethyl-guanidine (Gua). The starting library was modified via CuAAC with the four azides individually, pooled subsequently and used for the next SELEX cycle including selection with washing steps, elution of the DNA/protein complex and subsequent PCR. Here the modified DNA can be used as a template for the polymerase. Thus, the modification is removed and alkyne bearing DNA is amplified. This ds DNA is digested to ssDNA by λ-exonuclease (SSD) and the ssDNA is used for the click-modification. For the assignment of the sequences (single azide selection steps) to the respective modification needed for binding, the ssDNA is split into five samples, one remained unmodified, whereas the other four are click modified. This DNA is than individually used for the next two selection cycles.

The Split and combine click-SELEX reactions were performed as described above. Cycles 1-11 were done as multimodal selection and cycle 12 and 13 as single azide selections. Starting from the first cycle 1 to 11, 125 pmol of the respective click competitor was used (500 pmol in total) in cycles 12 and 13 500 pmol of the respective click competitor was added. Starting from cycle 2, DNA was incubated first with 50 µL empty beads as counter selection step. The selection details of the selection cycles are summarised in Table 3 below.

**Table 3: Overview of CXCL9 multimodal selection**

| cycle | DNA [pmol] | BSA [mg/mL] | salmon sperm DNA [mg/mL] | dextran sulfate [mg/ml] | CXCL9 beads [pmol] | incubation [min] | wash time | wash volume [µL] | PCR cycles |
|---|---|---|---|---|---|---|---|---|---|
| 1 | 4x125 | 0.2 | 0.1 | | 328 | 30 | 1x short 2x 3min | 100 | 8 |
| 2 | 31 | 0.2 | 0.1 | | 328 | 30 | 1x short 2x 3min | 100 | 10 |
| 3 | 18 | 0.6 | 0.1 | | 164 | 25 | 1x short 2x 5min | 150 | 12 |
| 4 | 18 | 0.6 | 0.25 | | 164 | 20 | 1x short 3x 5min | 200 | 11 |
| 5 | 10 | 0.6 | 0.25 | | 82 | 15 | 1x short 3x 7.5min | 250 | 12 |
| 6 | 15 | 1.2 | 0.1 | | 41 | 10 | 1x short 3x 10min | 300 | 15 |
| 7 | 14 | 1.2 | 0.1 | 0.01 | 6.6 | 5 | 1x short 5x 10min | 300 | 12 |
| 8 | 9 | 1.2 | 0.1 | 0.1 | 1.3 | 5 | 1x short 15x 5min | 500 | 15 |
| 9 | | 1.2 | 0.1 | 1 | 0.33 | 5 | 1x short 15x 5min | 400 | 14 |
| 10 | 11 | 1.2 | 0.1 | 1 | 0.007 | 5 | 1x short 15x 5min | 500 | 14 |
| 11 | 13 | 1.2 | 0.1 | 1 | 0.007 | 5 | 1x short 15x 5min | 600 | 14 |
| 12 E | 10 | 1.2 | 0.1 | 1 | 0.007 | 5 | 1x short 15x 5min | 600 | 18 |
| 12 In | 4 | 1.2 | 0.1 | 1 | 0.007 | 5 | 1x short 15x 5min | 600 | 12 |
| 12 Bn | 5 | 1.2 | 0.1 | 1 | 0.007 | 5 | 1x short 15x 5min | 600 | 14 |
| 12 Phe | 5 | 1.2 | 0.1 | 1 | 0.007 | 5 | 1x short 15x 5min | 600 | 12 |
| 12 Gua | 4 | 1.2 | 0.1 | 1 | 0.007 | 5 | 1x short 15x 5min | 600 | 18 |
| 13E | 12 | 1.2 | 0.1 | 1 | 0.007 | 5 | 1x short 15x 5min | 600 | 16 |
| 13 In | 10 | 1.2 | 0.1 | 1 | 0.007 | 5 | 1x short 15x 5min | 600 | 11 |
| 13 Bn | 7 | 1.2 | 0.1 | 1 | 0.007 | 5 | 1x short 15x 5min | 600 | 12 |
| 13 Phe | 12 | 1.2 | 0.1 | 1 | 0.007 | 5 | 1x short 15x 5min | 600 | 11 |
| 13 Gua | 8 | 1.2 | 0.1 | 1 | 0.007 | 5 | 1x short 15x 5min | 600 | 18 |

Interaction analysis was performed using flow cytometry as described above of the starting library binding to CXCL9 immobilised on beads and after 11 or 13 selection cycles. For this, 500 nM Cy-5 labelled DNA from the starting library (SL) as well as from selection cycle 11 and selection cycle 13 were incubated with CXCL9 immobilised on magnetic beads. DNA was either unmodified (E) or click modified with indole (In), benzyl (Bn), phenol (Phe) or guanidine (Gua) azide.

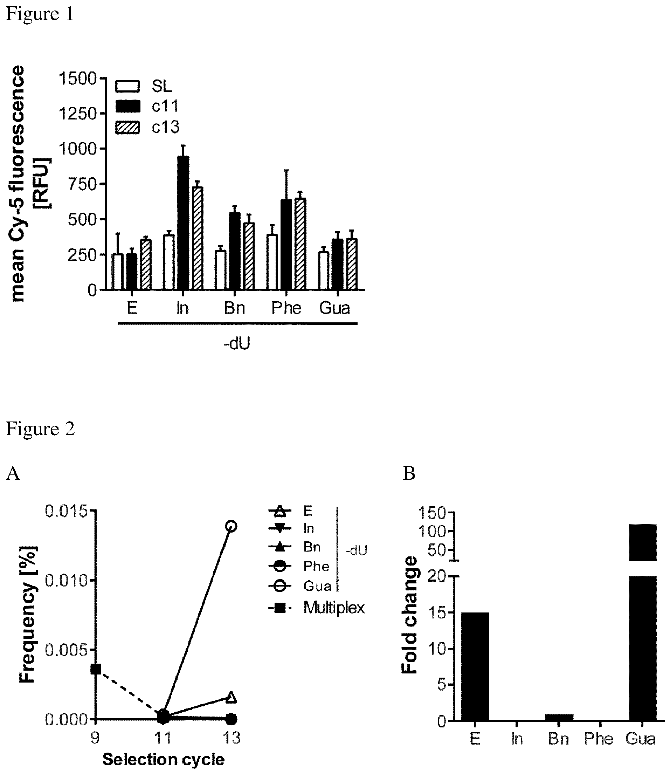

The figure 1 shows the results of the interaction analysis (n=2-4, mean ± SD). As can be taken from figure 1, after 11 and 13 selection cycles no binding of unmodified DNA was seen, but enhanced binding of cycle 11 compared to the starting library was seen with indole, benzyl and phenol modifications.

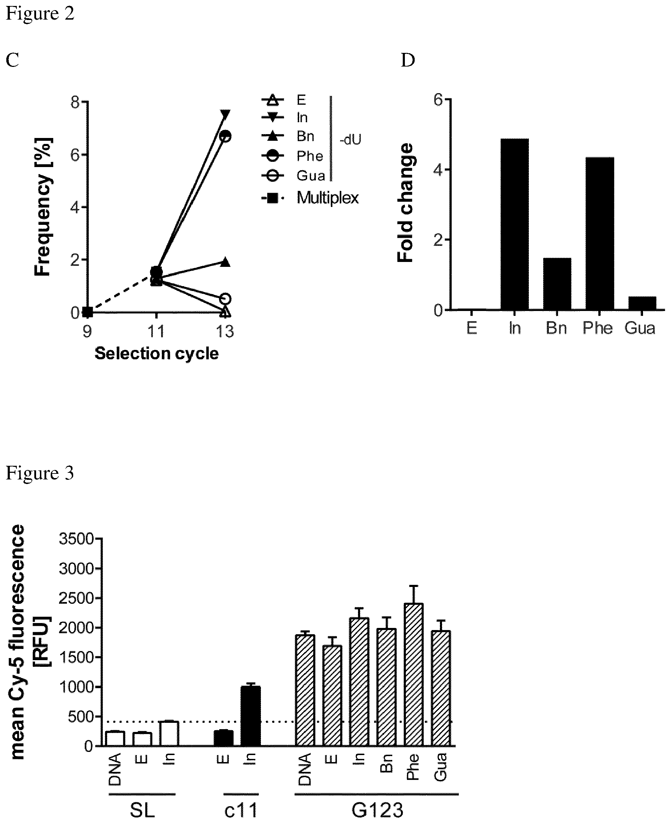

After 13 selection cycles, each of the obtained DNA libraries from selection cycles 0, 4, 7, 9,11 and 13 was analysed by next-generation sequencing (NGS) as described above. Figure 2 shows the NGS results of the two analysed sequences SEQ ID NO: 4, in the following denoted G123, and SEQ ID NO: 5, in the following denoted 129. Figures 2a) and 2b) show frequency and fold change, respectively, of cycles 9, 11 and 13 of the sequence G123. Figures 2c) and 2d) show frequency and fold change of cycles 9, 11 and 13 of sequence 129. As can be taken from figure 2, the sequences G123 (SEQ ID NO: 4) and 129 (SEQ ID NO: 5), were found to be enriched.

### Example 2

Determination of binding of the aptamers of SEQ ID NO: 4 and 6 to CXCL9 using flow cytometry

The interaction analysis with CXCL9 was performed using flow cytometry as described above. 500 nM Cy-5 labelled DNA from the starting library (SL) as well as from selection cycle 11 (c11) and the aptamer of SEQ ID NO: 4 and SEQ ID NO: 6 (G123) were incubated with CXCL9 immobilised on magnetic beads. The DNA used was either conventional DNA (DNA, SEQ ID NO: 6), unmodified E-dU (E) or click modified with indole (In), benzyl (Bn), phenol (Phe) or guanidine (Gua) azide. Figure 3 shows the fluorescence results of the aptamer of SEQ ID NO: 4 denoted G123 compared to the starting library (SL) and selection cycle 11 (c11) (n=2-4, mean ± SD). As can be seen in figure 3, the aptamer G123 showed binding to CXCL9 when modified with indole, benzyl, phenol and guanidine as well as in form of unmodified, conventional DNA (SEQ ID NO: 6).

### Example 3

Determination of affinity of the aptamers of SEQ ID NO: 4 and 5 to CXCL9 using ELONA

Affinity determination and the influence of modifications on the binding of the aptamer 129 to CXCL9 was further analysed using enzyme linked oligonucleotide assay (ELONA). Biotin labelled DNA was detected by colorimetric change using streptavidin-horseradish peroxidase (SA-HRP) as described above using 100 nM aptamer.

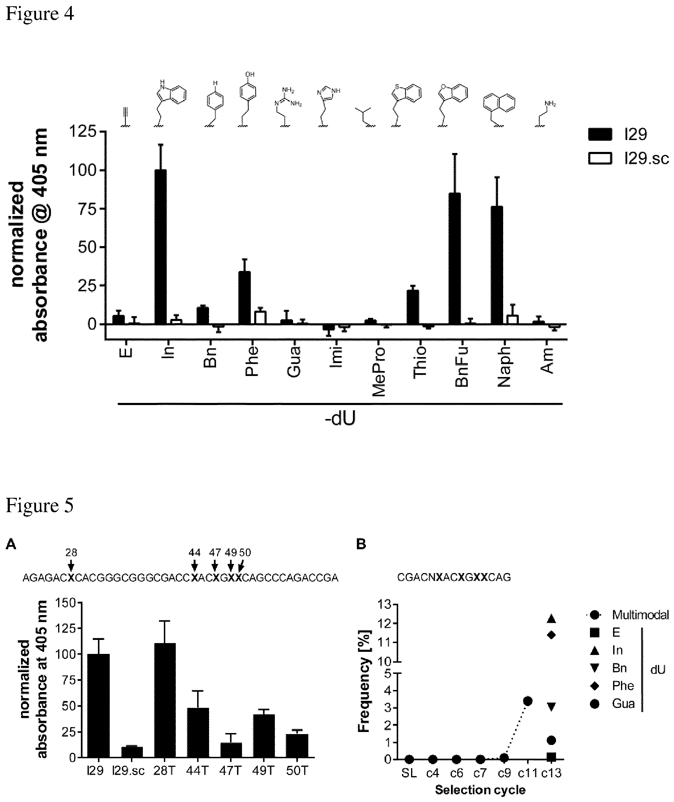

The influence of 10 different modifications on affinity of aptamer SEQ ID NO: 5 (129) was determined: indole (In), benzyl (Bn), phenol (Phe), guanidine (Gua), imidazole (Imi), methylpropane (MePro), benzothiophene (Thio), benzofurane (BnFu), naphthalene (Naph) and ethylamine (Am) or azide without modification (E). Figure 4 shows the results of the binding of 100 nM 129 with the 10 different modifications to CXCL9, where the binding was normalized to the binding with In-dU, (n=2, mean ± SD). As can be taken from Figure 4, binding of the aptamer 129 to CXCL9 requires indole, benzofurane, naphthalene, phenol or benzothiophene modifications.

In parallel, the influence of indole (In) modification and azide without modification (E) on the affinity of aptamer SEQ ID NO: 4 (G123) was determined using ELONA and compared to the affinity of unmodified DNA (DNA). The following table 4 summarises the binding of G123 and 129 and the affinity constants determined with ELONA (n=2, mean ± SD). In the table, "+" represents successful binding, while "-" means no detection of binding, "n.d." denotes not determined.

**Table 4: Binding and affinity constants of aptamer SEQ ID NO: 4 (G123) and aptamer SEQ ID NO: 5 (129) with different modifications**

| Sequence | Modification | Binding | KD [nM] |
|---|---|---|---|
| G123 | DNA | + | 92±14 |
| | E | + | n.d. |
| | In | + | 39±5 |
| | Bn | + | n.d. |
| | Phe | + | n.d. |
| | Gua | + | n.d. |
| I29 | E | - | |
| | In | + | 12±2 |
| | Phe | + | 177±28 |
| | BnFu | + | 17±4 |
| | Naph | + | 21±4 |
| | Thio | + | n.d. |
| | Gua | - | |
| | Imi | - | |
| | MePro | - | |
| | Am | - | |

These results show that two highly affine aptamers were selected, where the aptamer of SEQ ID NO: 5 (129) binds if modified with aromatic groups such as indole, while the aptamer of SEQ ID NO: 4 (G123) binds as DNA (SEQ ID NO: 6) but also with different modifications.

### Example 4

Determination of specificity of binding of aptamers of SEQ ID NO: 4 and 5 to CXCL9 and CXCL11

Binding of aptamers of SEQ ID NO: 4 (G123) and SEQ ID NO: 5 (129) to different proteins was determined using ELONA. Biotin labelled DNA (conc = 2x KD) of unmodified aptamer G123 (DNA, SEQ ID NO: 6), indole-modified aptamer G123 (G123 In) and indole-modified aptamer 129 (129 In) was detected by colorimetric change using streptavidin-horseradish peroxidase as described above. The pro-inflammatory chemokines CXCL9, CXCL10, CXCL11, and CXCL1 were used, as well as soluble interleukin-2 receptor (sCD25), C-reactive protein (CRP), C-C motif chemokine ligand 17 (CCL17), C-C motif chemokine ligand 22 (CCL22), C-C motif chemokine ligand 3(CCL3) and human serum albumin (HAS).

The following table 5 summarises the binding of unmodified aptamer G123 (DNA, SEQ ID NO: 6), indole-modified aptamer G123 (G123 In) and indole-modified aptamer 129 (129 In). In the table, "+" represents successful binding, while "-" means no detection of binding.

**Table 5: Binding of G123 and 129 to different proteins.**

| Protein | G123 DNA binding | G123 In binding | I29 In binding |
|---|---|---|---|
| CXCL9 | + | + | + |
| CXCL10 | - | - | - |
| CXCL11 | + | + | + |
| CXCL1 | - | - | - |
| sCD25 | - | - | - |
| CRP | - | - | - |
| CCL17 | - | - | - |
| CCL22 | - | - | - |
| CCL3 | - | - | - |
| HSA | - | - | - |

As can be seen from table 5, next to pro-inflammatory chemokine CXCL9 also CXCL11 is recognized by the aptamer G123, either unmodified or indole-modified, and indole-modified aptamer 129. The binding results shows that both aptamer of SEQ ID NO: 5 (129) and SEQ ID NO: 4 (G123) are highly selective aptamers, showing affinity to CXCL9, but no binding to CXCL10, although all three chemokines bind to the same receptor (CXCR3).

The following table 6 summarises the affinity constants of the binding of unmodified aptamer G123 (DNA, SEQ ID NO: 6), indole-modified aptamer G123 (G123 In) and indole-modified aptamer 129 (129 In) to pro-inflammatory chemokines CXCL9 and CXCL11 determined with ELONA (n=2, mean ± SD).

**Table 6: Affinity constants of G123 to CXCL11 and CXCL9**

| Sequence | KD CXCL11 [nM] | KD CXCL9 [nM] |
|---|---|---|
| G123 DNA | 249±44 | 92±14 |
| G123 In | 73±15 | 39±5 |
| 129 In | 17±2 | 12±2 |

The affinity constants show that two highly affine aptamers for pro-inflammatory chemokines CXCL9 and CXCL11 could be identified.

### Example 5

### Analysis of binding motif

### 5.1 Analysis of importance of thymidines for binding to CXCL9

The influence of substitutions of the nucleosides at positions 28, 44, 47, 49 and 50 of the aptamer of SEQ ID NO: 5 was analysed in view of their importance for binding of the aptamers to CXCL9. For this, in the aptamer sequence of SEQ ID NO: 5 (129) the Ed-U modifications were substituted with conventional Ts and binding to CXCL9 was determined using enzyme linked oligonucleotide assay (ELONA) where Biotin labelled DNA (100 nM) of 129 variants was detected by colorimetric change using streptavidin-horseradish peroxidase (SA-HRP) at 405 nm.

Figure 5A shows the effect of substitution of the Ed-U modifications with conventional thymidines (Ts) on binding to CXCL9 (n=2, mean ± SD). As can be seen in figure 5A, except for position 28, all modifications are important for binding to CXCL9. Further, it can be seen that the indole modification provides the highest stability. Figure 5B shows the frequency of the 129 motif (CGACNXACXGXXCAG (SEQ ID NO: 16); X=Ed-U, N=any base) enrichment during CXCL9 SELEX of example 1.

### 5.2 G-quadruplex characterisation

For further characterisation, a G-quadruplex characterisation of the sequence of the aptamer of SEQ ID NO: 4 (G123) was performed using flow cytometer binding assay. 100 nM Cy-5 labelled G123 was incubated with CXCL9 magnetic beads. DNA was either conventional DNA (DNA, SEQ ID NO: 6) or click modified with indole (In), benzyl (Bn), phenol (Phe) or guanidine (Gua) azide. Incubation was done in different buffer conditions, i.e. with kalium kation (K⁺) in the binding buffer, without kalium kation (w/o K⁺), or K⁺ and NH₄OAc (w/o K⁺ & NH₄OAc).

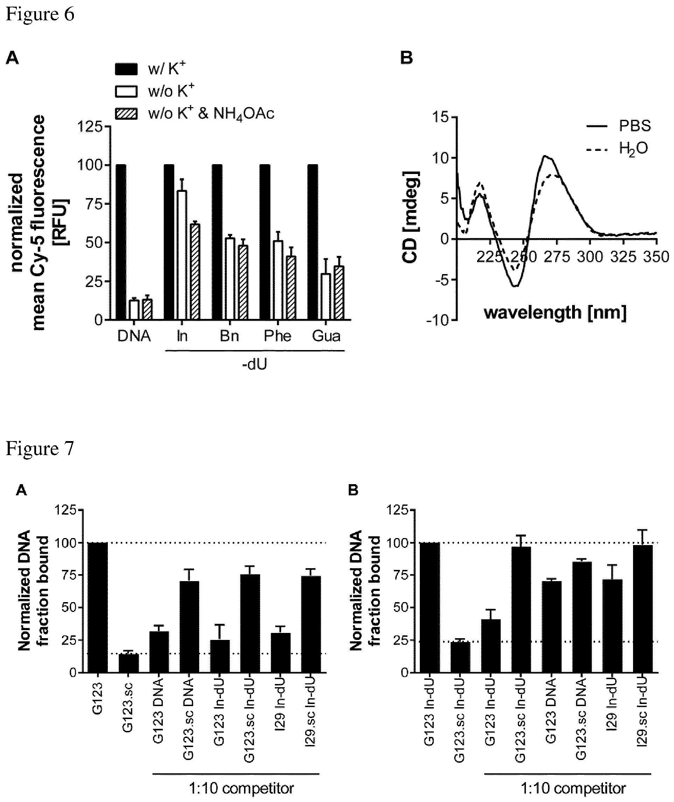

Figure 6A shows the results of the flow cytometer binding assay (n=2-5, mean ± SD, binding normalized to binding in SELEX conditions (w/ K⁺)). As can be seen in figure 6A, G123 DNA cannot bind without K⁺ in the binding buffer, whereas click modified DNA can still bind to CXCL9, even without K⁺ and NH₄OAc. Figure 6B shows the circular dichroism analysis of G123 DNA in water and in PBS. Figure 6B confirms the G-rich quadruplex motif. This shows that the G-rich motif of SEQ ID NO: 1 is important for binding to the pro-inflammatory chemokine CXCL9.

### Example 6

### Determination of binding to CXCL9 epitope using competition assays

The binding of indole-modified aptamer of SEQ ID NO: 5 (129 In), unmodified aptamer of SEQ ID NO: 6 (G123 DNA), and indole-modified aptamer of SEQ ID NO: 4 (G123 In) to pro-inflammatory chemokine CXCL9 was determined in competition assay. 100 nM Cy-5 labelled DNA was incubated simultaneous with 1µM unlabelled DNA and thus competed for binding. After incubation the remaining Cy5 labelled DNA on CXCL9-magnetic beads was analysed with flow cytometry as described above. As control, Cy5-labeled DNA without competitor was analysed and was used to normalize data.

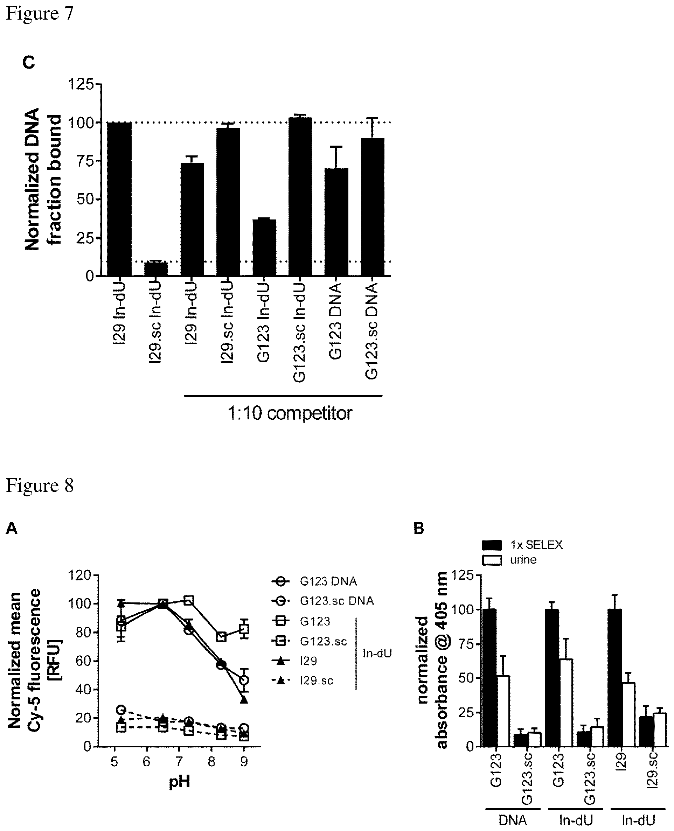

Figure 7 shows the results of the competition assay of sequence 129 In, G123 DNA and G123 In. Binding of Cy5 labelled scrambled sequence is used as non-binding control. (n=2, mean ± SD). As can be taken from figure 7A, competition of G123 DNA can be mainly seen for all 3 sequences (G123, G123 (In) and 129 (In) but also to some parts of the scrambled variants.

Figure 7B shows the competition results for unmodified aptamer of SEQ ID NO: 6 (G123 DNA). Figure 7C shows the competition results for modified aptamer of SEQ ID NO: 5 (129 In). As can be taken from figure 7C, competition of of 129 (In) can solely be seen for 129 (In), G123 (In) and G123.

This shows that the aptamers probably bind to the same epitope on CXCL9. In a diagnostic test for detecting the chemokine CXCL9, the aptamers may be used in a sandwich-complex with antibodies to CXCL9, which bind to different epitopes. Sandwich-complexes containing both aptamers G123 and 129 seem less useful as both aptamers compete for the binding epitope.

### Example 7

### Determination of binding conditions to CXCL9 in buffer and urine

### 7.1 Binding of aptamers to CXCL9 in buffers of different pH values

Binding of aptamers to CXCL9 in buffer of different pH values was characterised using flow cytometer binding assay. 100 nM Cy-5 labelled aptamers were incubated with CXCL9 magnetic beads in buffers of pH values of 5.2, 6.5, 7.3, 8.3 and 9.0. The aptamer sequences tested were either conventional aptamer of SEQ ID NO: 6 (G123 DNA), or the aptamer of SEQ ID NO: 4 click-modified with indole azide (G123), or the aptamer of SEQ ID NO: 5 click-modified with indole azide (129).

Figure 8A shows the binding to CXCL9 depending on buffer conditions (n=3-4, SD ± mean, binding normalized to binding in SELEX conditions (pH 6.5)). As can be seen in Figure 8A, binding of indole-modified G123 was more stable over a broad pH range, compared to unmodified G123 and indole-modified 129.

### 7.2 Binding of aptamers to CXCL9 in human urine

The binding of the conventional aptamer of SEQ ID NO: 6 (G123 DNA), the aptamer of SEQ ID NO: 4 click-modified with indole azide (G123 In-dU), and the aptamer of SEQ ID NO: 5 click-modified with indole azide (129 In-dU) to CXCL9 was further analysed in SELEX buffer of pH 6.5 and in untreated human urine from a healthy donor using ELONA binding assay of biotin labelled aptamers. Figure 8B shows the binding to CXCL9 in SELEX buffer of pH 6.5 and in human urine (n=2, mean ± SD, binding normalized to binding in SELEX buffer). As can be seen in Figure 8B, binding of all sequences was reduced in urine, but still was distinguishable from control sequences.

This shows that the aptamers are usable in human urine for diagnostic tests to detect the chemokines CXCL9 and CXCL11 in urine of kidney allograft patients.

### Example 8

### Determination of the binding of aptamers to endogenous CXCL9

The binding of the aptamers was further analysed in cell supernatant from human peripheral blood mononuclear cells. Cell supernatant was generated from isolated PBMC's (peripheral blood mononuclear cells) from blood donors that were differentiated for 7 days to macrophages. Macrophages were stimulated with Interferon-gamma (10 ng/ml) and LPS (lipopolysaccharide, 1 µg/ml) for 24-48 h in RPMI 1640 Medium + 5 % humane AB Serum + 1 % Penicillin/Streptomycin + 25 ng/ml m-CSF. Cell supernatant was collected and directly stored at -80°C.

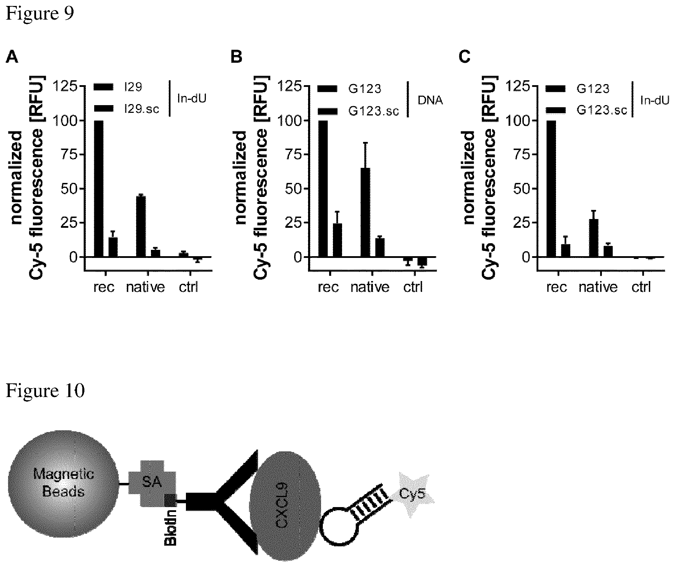

The assay was performed as sandwich assay. CXCL9 specific antibody was immobilized on magnetic beads. This antibody-protein-beads complex were incubated with recombinant CXCL9 in control cell supernatant (rec) and native CXCL9 (native) or control supernatant without CXCL9 (ctrl) in cell supernatant and analysed with flow cytometry. The CXCL9 concentration used in this assay was 0.9 nM (11 ng/ml) which is lower than the CXCL9 concentration in urine of patients with renal rejection that was reported to be 14 nM (178 ng/ml). After washing, 150 nM Cy-5 labeled DNA was used to detect the resulting complex as depicted in Figure 10.

The binding of 150 nM of the respective Cy-5 labeled aptamers of SEQ ID NO: 5 click-modified with indole azide (129 In-dU), the unmodified, conventional aptamer of SEQ ID NO: 6 (G132 DNA) and the aptamer of SEQ ID NO: 4 click-modified with indole azide (G123 In-dU) was tested.

Figure 9 shows the results of the binding of CXCL9 aptamers to endogenous CXCL9 for native CXCL9 in cell supernatant (native), recombinant CXCL9 in control cell supernatant (rec) and control supernatant without CXCL9 (ctrl) with Cy5-labelled 129 In-dU in Figure 9A, G132 DNA in Figure 9B and G123 In-dU in Figure 9C. Binding was normalized to recombinant protein in control supernatant (n=2, mean ± SD). As can be taken from figure 9, all aptamers can bind the endogenous CXCL9, even if binding was reduced compared to recombinant CXCL9.

## Claims

1. An aptamer comprising a nucleotide sequence 5'-N₁N₂GN₃CCN₁AN₄N₅N₁N₆N₇N₈N₁-3' (SEQ ID NO: 1) or pharmaceutically acceptable salts thereof, wherein:
N₁ represents T or 5-ethynyl-2'-deoxyuridine (EdU) which is modified with a side chain selected from the group comprising indole, benzofurane, naphthalene, phenol, benzothiophene, guanidine and benzyl,
N2 represents a sequence of 9 or 11 contiguous nucleotides comprising at least 5 guanidine nucleotides,
N₃ represents A or C,
N₄ represents A or a deletion,
N₅ represents C or G,
N₆ represents C or a deletion,
N₇ represents C or G,
N₈ represents G or N₁.

2. The aptamer according to claim 1, wherein the aptamer comprises or consists of a nucleotide sequence 5'-CACGACGCAAGGGACCACAGGGAGGGAGGGN₁GGGC AAAGGGCCCN₁AAGN₁CCGN₁AACAAAAACACAGCACGACACCGCAGAGGC A-3' (SEQ ID NO: 4) or pharmaceutically acceptable salts thereof, wherein N₁ represents T or EdU which is modified with a side chain selected from the group comprising indole, phenol, guanidine and benzyl.

3. The aptamer according to according to claim 1 or 2, wherein the aptamer comprises or consists of a nucleotide sequence 5'-CACGACGCAAGGGACCACAGGGAGGGA GGGTGGGCAAAGGGCCCTAAGTCCGTAACAAAAACACAGCACGACACCGC AGAGGCA-3' (SEQ ID NO: 6) or a nucleotide sequence SEQ ID NO: 4 wherein N₁ represents EdU which is modified with indole, or pharmaceutically acceptable salts thereof.

4. The aptamer according to claim 1, wherein the aptamer comprises or consists of a nucleotide sequence 5'- CACGACGCAAGGGACCACAGGAGAGACN₁CACGGG CGGGCGACCN'₁ACN'₁GN'₁N'₁CAGCCCAGACCGACAGCACGACACCGCAGA GGCA-3' (SEQ ID NO: 5) or pharmaceutically acceptable salts thereof, wherein N₁ represents T or EdU which is modified with an aromatic group selected from the group comprising indole, benzofurane, naphthalene, phenol and benzothiophene and and N'₁ represents EdU which is modified with an aromatic group selected from the group comprising indole, benzofurane, naphthalene, phenol and benzothiophene.

5. The aptamer according to claim 4, wherein the aromatic group is selected from indole, benzofurane or naphthalene.

6. An aptamer comprising or consisting of a nucleotide sequence selected from the group of SEQ ID NO: 1-9 or pharmaceutically acceptable salts thereof, for use as a medicament or a diagnostic reagent.

7. The aptamer for use according to claim 6, wherein the aptamer is for use in the detection or diagnosing of a rejection of a renal allograft.

8. The aptamer for use according to claim 6 or 7, wherein the aptamer is used in form of a complex with an antibody against chemokines CXCL9 or CXCL11.

9. A diagnostic composition comprising as an active ingredient an aptamer comprising or consisting of a nucleotide sequence selected from the group of SEQ ID NO: 1-9 or pharmaceutically acceptable salts thereof.

10. The diagnostic composition of claim 9, for use in the detection or diagnosing of a rejection of a renal allograft.

11. A pharmaceutical composition comprising as an active ingredient an aptamer comprising or consisting of a nucleotide sequence selected from the group of SEQ ID NO: 1-9 or pharmaceutically acceptable salts thereof.

12. Use of an aptamer comprising or consisting of a nucleotide sequence selected from the group of SEQ ID NO: 1-9 or pharmaceutically acceptable salts thereof for the manufacture of a medicament or a diagnostic reagent, particularly for use in the detection or diagnosing of a rejection of a renal allograft.

13. An *in vitro* method of detecting or diagnosing a rejection of a renal allograft, the method comprising the step of detecting the binding of an aptamer comprising or consisting of a nucleotide sequence selected from the group of SEQ ID NO: 1-9 or pharmaceutically acceptable salts thereof to CXCL9 or CXCL11 in a sample obtained from a subject.

14. The method of claim 13, wherein the sample is selected from urine or serum of a renal allograft patient.
